(19) Europäisches Patentamt
European Patent Office
Office européen des brevets

(11) Veröffentlichungsnummer: **0 065 780**
**A2**

(12) # EUROPÄISCHE PATENTANMELDUNG

(21) Anmeldenummer: 82104524.2

(22) Anmeldetag: 24.05.82

(51) Int. Cl.³: **C 07 C 109/06**
C 07 C 109/12, C 07 C 133/00
C 07 C 143/825, C 07 C 159/00
C 07 C 161/00, A 01 N 39/00
A 01 N 47/24, A 01 N 47/42
A 01 N 51/00

(30) Priorität: 26.05.81 CH 3441/81
31.03.82 CH 1983/82

(43) Veröffentlichungstag der Anmeldung: 01.12.82 Patentblatt 82/48

(84) Benannte Vertragsstaaten: AT BE CH DE FR GB IT LI NL SE

(71) Anmelder: F. HOFFMANN-LA ROCHE & CO. Aktiengesellschaft

CH-4002 Basel(CH)

(72) Erfinder: Fráter, Georg, Dr.
Am Pfisterhölzli 22
CH-8606 Greifensee(CH)

(72) Erfinder: Suchy, Milos, Dr.
Grossplatzstrasse 3
CH-8122 Pfaffhausen(CH)

(72) Erfinder: Wenger, Jean, Dr.
Brunnenwiesenstrasse 1
CH-8610 Uster(CH)

(72) Erfinder: Winternitz, Paul, Dr.
Am Pfisterhölzli 50
CH-8606 Greifensee(CH)

(74) Vertreter: Lederer, Franz, Dr. et al,
Patentanwälte Dr. Lederer Franz Meyer-Roxlau Reiner F.
Lucile-Grahn-Strasse 22
D-8000 München 80(DE)

(54) **Propionsäureamide, Herstellung dieser Verbindungen, Unkrautbekämpfungsmittel, die diese Verbindungen als Wirkstoffe enthalten, sowie Verwendung solcher Verbindungen und Mittel zur Bekämpfung von Unkräutern.**

(57) Die Erfindung betrifft Propionsäureamide der Formel

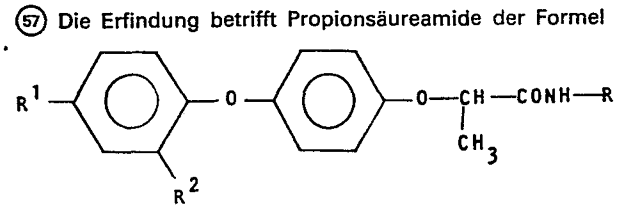

1

worin $R^1$, $R^2$ und R die in der Beschreibung angegebenen Bedeutungen besitzen, Verfahren zu deren Herstellung, Unkrautbekämpfungsmittel, die diese Verbindungen als Wirkstoffe enthalten, sowie die Verwendung solcher Verbindungen bzw. Mittel zur Bekämpfung von Unkräutern.

EP 0 065 780 A2

0065780

F.Hoffmann-La Roche & Co.Aktiengesellschaft, Basel/Schweiz

24. Mai 1982

RAN 6102/28

Propionsäureamide, Herstellung dieser Verbindungen, Unkrautbekämpfungsmittel, die diese Verbindungen als Wirkstoffe enthalten, sowie Verwendung solcher Verbindungen und Mittel zur Bekämpfung von Unkräutern.

Die Erfindung betrifft Propionsäureamide der allgemeinen Formel

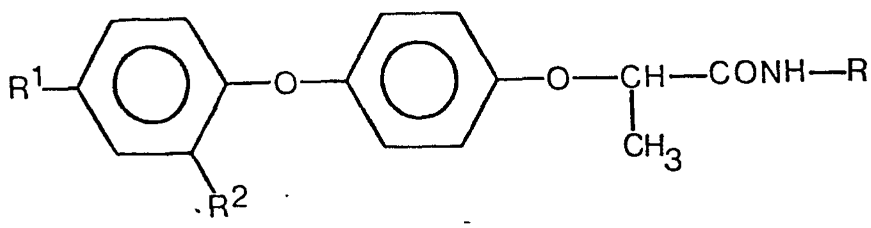

I

worin $R^1$ Chlor, Jod oder Trifluormethyl,

$R^2$ Wasserstoff oder Chlor

und R eine der folgenden Gruppen (a) - (e) bedeuten:

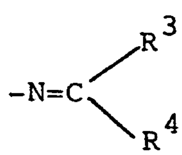

(a)

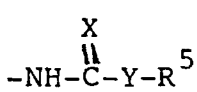

(b)

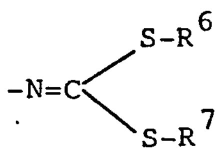

(c)

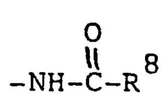

(d)

$-NH-SO_2-R^9$ (e)

Pa/30.3.82

wobei in den obigen Formeln der Gruppen (a) - (e) $R^3$ und $R^4$ Wasserstoff; $C_{1-4}$-Alkyl; $C_{1-4}$-Halogenalkyl; Phenyl, gegebenenfalls substituiert mit Chlor, Methyl, Methoxy und/oder Nitro; oder Benzyl, gegebenenfalls substituiert im Arylkern mit Chlor, Methyl, Methoxy und/oder Nitro, bedeuten oder zusammen mit dem Kohlenstoffatom, an das sie geknüpft sind, einen $C_{5-7}$-Cycloalkanring bilden,

$R^5$ $C_{1-4}$-Alkyl, $C_{2-4}$-Alkenyl, $C_{2-4}$-Alkinyl, Phenyl oder Benzyl bedeutet,

X und Y Sauerstoff oder Schwefel bedeuten,

$R^6$ und $R^7$ $C_{1-4}$-Alkyl, $C_{2-4}$-Alkenyl, $C_{2-4}$-Alkinyl, Phenyl oder Benzyl bedeuten oder zusammen eine $C_{2-4}$-Alkylengruppe bilden, mit der Massgabe, dass $R^6$ und $R^7$ nicht gleichzeitig Phenyl bedeuten,

$R^8$ Wasserstoff; $C_{1-4}$-Alkyl; $C_{1-4}$-Halogenalkyl; $C_{2-4}$-Alkenyl; oder Phenyl, gegebenenfalls substituiert mit Chlor, Methyl, Methoxy und/oder Nitro, bedeutet

und $R^9$ $C_{1-4}$-Alkyl; oder Phenyl, gegebenenfalls substituiert mit Chlor, Methyl, Methoxy und/oder Nitro, bedeutet.

Die Verbindungen der Formel I besitzen herbizide Eigenschaften und eignen sich somit als Unkrautbekämpfungsmittel oder als Wirkstoffe von Unkrautbekämpfungsmitteln. Somit umfasst die Erfindung auch Unkrautbekämpfungsmittel, die mindestens eine Verbindung der Formel I als Wirkstoff enthalten, ein Verfahren zur Herstellung dieser Verbindungen sowie die Verwendung solcher Verbindungen bzw. Mittel zur Bekämpfung von Unkräutern.

In der obigen Formel I sind unter "$C_{1-4}$-Alkyl" sowohl geradkettige als auch verzweigte Alkylgruppen zu verstehen, wie Methyl, Aethyl, n-Propyl, Isopropyl, n-Butyl, sek.Butyl, Isobutyl und tert.Butyl. Dies gilt auch für die $C_{1-4}$-

Alkylgruppe enthaltende Halogenalkylgruppe. Der Ausdruck "Halogen" in der letztgenannten Gruppe umfasst Fluor, Chlor, Brom und Jod. In diesem Zusammenhang umfasst der Ausdruck "Halogenalkyl" mit einem oder mehreren Halogenatomen substituiertes Alkyl, wobei mehrere Halogenatome gleich oder verschieden sein können. Wenn in einem substituierten Phenyl- oder Benzylrest zwei oder mehr Substituenten vorhanden sind, können die Substituenten gleich oder verschieden sein. In den Gruppen (a), (b) und (c) sind die Bedeutungen von $R^3$ und $R^4$; X und Y; bzw. $R^6$ und $R^7$ unabhängig voneinander.

Da mindestens ein asymmetrisches Kohlenstoffatom im Molekül vorhanden ist, können die Verbindungen der Formel I in optisch aktiver Form auftreten. Durch das Vorliegen der Stickstoff-Kohlenstoff-Doppelbindung in den Verbindungen der Formel I, worin R eine Gruppe (a) oder (c) bedeutet, tritt für jene Verbindungen, worin $R^3$ und $R^4$ bzw. $R^6$ und $R^7$ unterschiedliche Bedeutung haben, zusätzlich geometrische Isomerie auf. Solche geometrischen Isomeren werden als syn- und anti-Formen bezeichnet. Unabhängig davon kommt auch in gewissen Fällen eine Atropisomerie vor. Die Formel I soll demnach die Racemate sowie all diese möglichen isomeren Formen umfassen.

$R^1$ bedeutet vorzugsweise Jod oder Trifluormethyl.

$R^2$ bedeutet vorzugsweise Chlor.

$R^3$ und $R^4$, unabhängig voneinander, bedeuten vorzugsweise Wasserstoff, $C_{1-4}$-Alkyl, $C_{1-4}$-Halogenalkyl oder gegebenenfalls wie oben ausgeführt substituiertes Phenyl.

$R^5$ bedeutet vorzugsweise $C_{1-4}$-Alkyl.

X und Y, unabhängig voneinander, bedeuten vorzugsweise Sauerstoff.

$R^6$ und $R^7$, unabhängig voneinander, bedeuten vorzugsweise $C_{1-4}$-Alkyl, $C_{2-4}$-Alkenyl oder $C_{2-4}$-Alkinyl.

$R^8$ bedeutet vorzugsweise $C_{1-4}$-Alkyl, $C_{1-4}$-Halogenalkyl oder gegebenenfalls wie oben ausgeführt substituiertes Phenyl.

$R^9$ bedeutet vorzugsweise gegebenenfalls wie oben ausgeführt substituiertes Phenyl.

Im allgemeinen bedeutet R vorzugsweise eine Gruppe (a), (b), (c) oder (d).

Besonders bevorzugt sind die D-Formen der Verbindungen der Formel I.

Bevorzugte Verbindungen der Formel I sind:

1-Isopropyliden-2-{D-2-[p-(α,α,α-trifluor-p-tolyloxy)-phenoxy]-propionyl}-hydrazin,
1-Isopropyliden-2-{D-2-[p-(o-chlor-p-jodphenoxy)-phenoxy]-propionyl}-hydrazin,
1-Isopropyliden-2-{D-2-[p-(o-chlor-α,α,α-trifluor-p-tolyloxy)phenoxy]-propionyl}-hydrazin und
Aethyl-3-{D-2-[p-(o-chlor-α,α,α-trifluor-p-tolyloxy)-phenoxy]-propionyl}-carbazat.

Das erfindungsgemässe Verfahren zur Herstellung der Verbindungen der Formel I ist dadurch gekennzeichnet, dass man

a) eine Säure der Formel

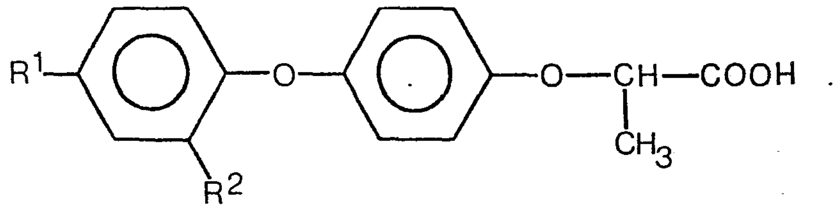

II

worin $R^1$ und $R^2$ die oben angegebenen Bedeutungen besitzen,

oder ein reaktionsfähiges Derivat davon, wie ein Halogenid, z.B. das Chlorid, oder das Anhydrid, mit einem Hydrazinderivat der Formel

$$H_2N-R \qquad III$$

worin R die oben angegebenen Bedeutungen besitzt,

umsetzt,

b) ein Hydrazid der Formel

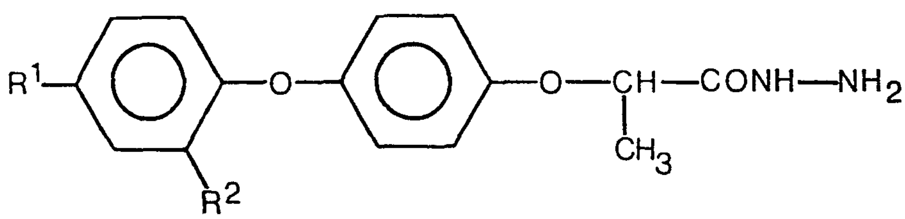

IV

worin $R^1$ und $R^2$ die oben angegebenen Bedeutungen besitzen,

mit einer Säure der Formel

$$HO-\overset{X}{\overset{\|}{C}}-Y-R^5 \qquad Va$$

$$HO-CO-R^8 \qquad Vb$$

$$HO-SO_2-R^9 \qquad Vc$$

worin $R^5$, X, Y, $R^8$ und $R^9$ die oben angegebenen Bedeutungen besitzen,

oder einem reaktionsfähigen Derivat davon, z.B. einem Halogenid oder dem Anhydrid, umsetzt,

c) eine Dithiocarbazinsäure der Formel

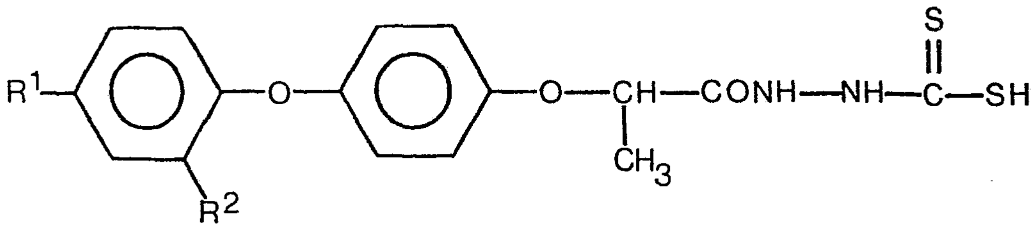

VI

worin $R^1$ und $R^2$ die oben angegebenen Bedeutungen besitzen,

oder ein Alkalimetall- oder Erdalkalimetallsalz davon, zweckmässigerweise in Gegenwart einer Base bzw. eines säurebindenden Mittels, mit einer Verbindung der Formel

$R^5$-$Z^1$ VII

worin $R^5$ die oben angegebenen Bedeutungen besitzt und $Z^1$ eine Abgangsgruppe, z.B. Halogen oder ein Aequivalent einer Sulfatgruppe, bedeutet,

umsetzt,

d) ein Dithiocarbazat der Formel

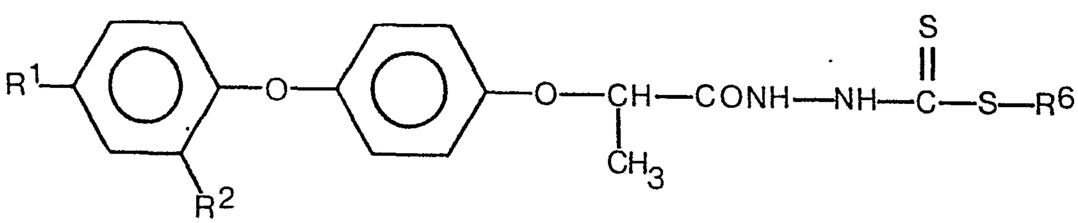

VIII

worin $R^1$, $R^2$ und $R^6$ die oben angegebenen Bedeutungen besitzen,

oder ein Alkalimetall- oder Erdalkalimetallsalz davon, zweckmässigerweise in Gegenwart einer Base, mit einer Verbindung der Formel

$R^7Z^1$ IX

worin $R^7$ und $Z^1$ die oben angegebenen Bedeutungen besitzen,

umsetzt,

e) ein Hydrazid der Formel IV, wie oben definiert, mit einem Aldehyd bzw. Keton der Formel

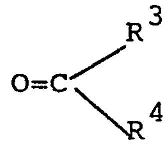

X

worin $R^3$ und $R^4$ die oben angegebenen Bedeutungen besitzen,

umsetzt, oder

f) ein Phenol der Formel

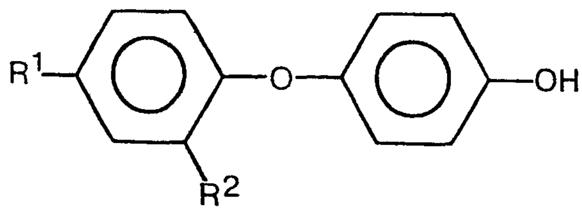

XI

worin $R^1$ und $R^2$ die oben angegebenen Bedeutungen besitzen,

oder ein Alkalimetallsalz davon, zweckmässigerweise in Gegenwart einer Base bzw. eines säurebindenden Mittels, mit einem Hydrazinderivat der Formel

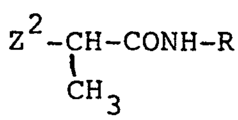

XII

worin R die oben angegebenen Bedeutungen besitzt, und $Z^2$ eine Abgangsgruppe, insbesondere Chlor, Brom, Jod, Mesyloxy oder Tosyloxy, bedeutet,

umsetzt.

Beispiele von in diesen Verfahrensvarianten verwendbaren Alkalimetall- bzw. Erdalkalimetallsalzen der entsprechenden Ausgangsmaterialien der Formeln VI, VIII und XI sind die Natrium-, Kalium-, Calcium- und Magnesiumsalze, wobei die Kalium- und, insbesondere, die Natriumsalze bevorzugt sind.

Bei der Verfahrensvariante a) handelt es sich um eine Amidierung, die nach an sich bekannten Methoden durchgeführt werden kann. Die Umsetzung wird also vorzugsweise in einem inerten Lösungsmittel, bei Temperaturen zwischen 0°C und der Rückflusstemperatur des Reaktionsgemisches, insbesondere bis 50°C, und in Gegenwart einer Base bzw. eines säurebindenden Mittels durchgeführt. Es kommen als geeignete Lösungsmittel vorzugsweise inerte organische Lösungsmittel in Frage, insbesondere Kohlenwasserstoffe, z.B. Benzol, Toluol und Xylole; chlorierte Kohlenwasserstoffe, z.B. Dichlormethan, Chloroform und Tetrachlormethan; Aether und ätherartige Verbindungen, z.B. Diäthyläther und Tetrahydrofuran; und Dimethylformamid. Zu den geeigneten Basen bzw. säurebindenden Mitteln gehören anorganische Basen, z.B. Alkalimetall- und Erdalkalimetallcarbonate und -bicarbonate, sowie organische Basen, z.B. tertiäre Amine, insbesondere Triäthylamin und Pyridin.

Die Verfahrensvariante b) führt zu jenen Verbindungen der Formel I, worin R eine Gruppe (b), (d) oder (e) bedeutet. Diese Variante kann nach an sich bekannten Methoden durchgeführt werden. Die Umsetzung wird vorzugsweise unter den Reaktionsbedingungen durchgeführt, die im Zusammenhang mit der Verfahrensvariante a) oben angegeben sind.

Bei der Verfahrensvariante c) handelt es sich um die Herstellung jener Verbindungen der Formel I, worin R eine Gruppe (b) und X und Y beide Schwefel bedeuten. Die Umsetzung erfolgt zweckmässigerweise in einem inerten Lösungsmittel, wie einem niederen Alkanol; Wasser; einem Aether oder einer ätherartigen Verbindung, z.B. 1,2-Dimethoxyäthan, Tetrahydrofuran oder Dioxan; Dimethylformamid; N-Methylpyrrolidon; oder Dimethylsulfoxid, bei Temperaturen zwischen 0°C und der Rückflusstemperatur des Reaktionsgemisches, insbesondere zwischen 20°C und 50°C. Als besonders geeignete Lösungsmittel für diesen Zweck erweisen sich Gemische der oben aufgeführten organischen Lösungsmittel mit Wasser. Falls das Ausgangsmaterial der Formel VI nicht

bereits in Form eines Salzes, nämlich eines Alkalimetall- oder Erdalkalimetallsalzes, verwendet wird, führt man die Umsetzung zweckmässigerweise in Gegenwart einer Base bzw. eines säurebindenden Mittels durch. Es eignen sich hierfür anorganische Basen, z.B. Alkalimetall- und Erdalkalimetallhydroxide und -carbonate sowie organische Basen, z.B. tertiäre Amine, wie Triäthylamin und Pyridin.

Das in der Verfahrensvariante c) verwendete Ausgangsmaterial der Formel VI kann zweckmässigerweise in situ hergestellt werden, indem man ein Hydrazid der Formel IV, wie oben definiert, mit Schwefelkohlenstoff, zweckmässigerweise unter den im Zusammenhang mit dieser Verfahrensvariante oben angegebenen Reaktionsbedingungen, umsetzt.

Die Verfahrensvariante d) führt zu jenen Verbindungen der Formel I, worin R eine Gruppe (c) bedeutet. Die Umsetzung wird vorzugsweise unter den gleichen Reaktionsbedingungen durchgeführt, wie für die Verfahrensvariante c) oben beschrieben sind.

Die Verfahrensvariante e), die zu jenen Verbindungen der Formel I führt, worin R eine Gruppe (a) bedeutet, wird zweckmässigerweise so durchgeführt, dass man die Ausgangsmaterialien der Formeln IV und X, gegebenenfalls in Gegenwart eines sauren Katalysators, z.B. p-Toluolsulfonsäure, in einem inerten organischen Lösungsmittel umsetzt. Es kommen als geeignete Lösungsmittel vorzugsweise Kohlenwasserstoffe, z.B. Benzol, Toluol und Xylole; chlorierte Kohlenwasserstoffe, z.B. Dichlormethan, Chloroform und Tetrachlormethan; und Aether und ätherartige Verbindungen, z.B. Diäthyläther, Tetrahydrofuran und Dioxan, in Frage. Das Ausgangsmaterial der Formel X kann selbst in gewissen Fällen vorteilhaft auch als das Lösungsmittel verwendet werden. Die Umsetzung erfolgt vorzugsweise in einem Temperaturbereich zwischen der Raumtemperatur und der Rückflusstemperatur des Reaktionsgemisches, insbesondere zwischen 20°C und 50°C.

Bei der Verfahrensvariante f) wird die Umsetzung zweckmässigerweise in basischem Medium durchgeführt. Zu diesem Zweck kommen anorganische Basen, z.B. Natrium- und Kaliumhydroxid und Natriumcarbonat und -bicarbonat, sowie organische Basen, z.B. tertiäre Amine, insbesondere Triäthylamin und Pyridin, in Frage. In vielen Fällen ist es zweckmässig, die Umsetzung in Gegenwart eines inerten organischen Lösungsmittels, wie eines Aethers oder einer ätherartigen Verbindung, z.B. Diäthyläther, Tetrahydrofuran oder Dioxan; Dimethylformamid; N-Methylpyrrolidon; oder Hexamethylphosphorsäuretriamid, durchzuführen. Die geeigneten Reaktionstemperaturen liegen im allgemeinen zwischen Raumtemperatur und der Rückflusstemperatur des Reaktionsgemisches, vorzugsweise zwischen 20°C und 50°C.

In jedem Fall kann das erhaltene Produkt nach an sich bekannten Methoden isoliert und gereinigt werden.

Sofern keine gezielte Synthese zur Isolierung reiner Isomerer durchgeführt wird, fällt das Produkt normalerweise als Gemisch zweier oder mehrerer Isomerer an. Die Isomeren können nach an sich bekannten Methoden aufgetrennt werden, wie z.B. in Industrial and Engineering Chemistry 60 (8), 12-28 beschrieben. Gewünschtenfalls können die optisch aktiven Isomeren auch durch Synthese aus entsprechenden optisch aktiven Ausgangsmaterialien hergestellt werden.

Die Ausgangsmaterialien der Formeln II, III, IV, Va, Vb, Vc, VII, IX, X, XI und XII sowie reaktionsfähige Derivate der Säuren der Formeln II, Va, Vb und Vc und Alkalimetallsalze der Phenole der Formel XI sind entweder bekannt oder können in an sich bekannter Weise hergestellt werden. Die Ausgangsmaterialien (Dithiocarbazinsäuren) der Formel VI können z.B. wie im Zusammenhang mit der Verfahrensvariante c) oben beschrieben hergestellt werden. Bei den Ausgangsmaterialien der Formel VIII handelt es sich gleichzeitig um Endprodukte der Formel I, worin R eine Gruppe (b) und X und Y beide Schwefel bedeuten und $R^5$ die

Bedeutungen von $R^6$ besitzt. Solche Ausgangsmaterialien können z.B. nach den oben angegebenen Verfahrensvarianten a), b), c) und f) von entsprechenden Ausgangsmaterialien hergestellt werden, und deren Alkalimetall- und Erdalkalimetallsalze können in an sich bekannter Weise hergestellt werden.

Die Verbindungen der Formel I besitzen herbizide Eigenschaften und eignen sich besonders zur Bekämpfung von Ungräsern, insbesondere von Hirsearten, Flughafer, Ackerfuchsschwanz, Quecke und Aleppohirse in allen gebräuchlichen Kulturen von Dikotylen, wie Soya-, Baumwolle-, Zuckerrüben-, Kartoffel- und Rapskulturen, in Reiskulturen sowie in Getreidekulturen. Besonders bevorzugt eignen sich die erfindungsgemässen Verbindungen zur Bekämpfung von Ungräsern in Soya-, Baumwolle-, Zuckerrüben- und Reiskulturen.

Im allgemeinen genügt eine Konzentration von 0,1 bis 2,0 kg Wirkstoff der Formel I/ha, vorzugsweise 0,25 bis 1,0 kg Wirkstoff der Formel I/ha, um den gewünschten herbiziden Effekt zu erzielen.

Die Verbindungen der Formel I sind sowohl Vorauflauf-Herbizide als auch Nachauflauf-Herbizide.

Das erfindungsgemässe Unkrautbekämpfungsmittel ist dadurch gekennzeichnet, dass es eine wirksame Menge mindestens einer Verbindung der Formel I, wie oben definiert, sowie Formulierungshilfsstoffe enthält. Das Mittel enthält zweckmässigerweise zumindest einen der folgenden Formulierungshilfsstoffe: feste Trägerstoffe; Lösungs- bzw. Dispersionsmittel; Tenside (Netz- und Emulgiermittel); Dispergatoren (ohne Tensidwirkung); und Stabilisatoren. Unter Verwendung solcher und anderer Hilfsstoffe können die Verbindungen der Formel I, also die herbiziden Wirkstoffe, in die üblichen Formulierungen übergeführt werden, wie Stäube, Pulver, Granulate, Lösungen, Emulsionen,

Suspensionen, emulgierbare Konzentrate, Pasten und dergleichen.

Die Verbindungen der Formel I sind im allgemeinen wasserunlöslich und können nach den für wasserunlösliche Verbindungen üblichen Methoden unter Verwendung der diesbezüglichen Formulierungshilfsstoffe konfektioniert werden. Die Herstellung der Mittel kann in an sich bekannter Weise durchgeführt werden, z.B. durch Vermischen des jeweiligen Wirkstoffes mit festen Trägerstoffen, durch Auflösen oder Suspendieren in geeigneten Lösungs- bzw. Dispersionsmitteln, eventuell unter Verwendung von Tensiden als Netz- oder Emulgiermitteln und/oder von Dispergatoren, durch Verdünnen bereits vorbereiteter emulgierbarer Konzentrate mit Lösungs- bzw. Dispersionsmitteln usw.

Als feste Trägerstoffe kommen im wesentlichen in Frage: natürliche Mineralstoffe, wie Kreide, Dolomit, Kalkstein, Tonerden und Kieselsäure und deren Salze (beispielsweise Kieselgur, Kaolin, Bentonit, Talkum, Attapulgit und Montmorillonit); synthetische Mineralstoffe, wie hochdisperse Kieselsäure, Aluminiumoxid und Silikate; organische Stoffe, wie Cellulose, Stärke, Harnstoff und Kunstharze; und Düngemittel, wie Phosphate und Nitrate, wobei solche Trägerstoffe z.B. als Pulver oder als Granulate vorliegen können.

Als Lösungs- bzw. Dispersionsmittel kommen im wesentlichen in Frage: Aromaten, wie Benzol, Toluol, Xylole und Alkylnaphthaline; chlorierte Aromaten und chlorierte aliphatische Kohlenwasserstoffe, wie Chlorbenzole, Chloräthylene und Methylenchlorid; aliphatische Kohlenwasserstoffe, wie Cyclohexan und Paraffine, z.B. Erdölfraktionen; Alkohole, wie Butanol und Glykol sowie deren Aether und Ester; Ketone, wie Aceton, Butan-2-on, Methylisobutylketon und Cyclohexanon; und stark polare Lösungs- bzw. Dispersionsmittel, wie Dimethylformamid, N-Methylpyrrolidon und Dimethylsulfoxid, wobei solche Lösungs- bzw. Dispersionsmittel vorzugsweise Flammpunkte von mindestens 30°C und Siedepunkte von mindestens 50°C aufweisen, und Wasser. Unter den Lösungs- bzw. Dispersionsmitteln kommen auch in Frage sogenannte verflüssigte gasförmige Streckmittel oder Trägerstoffe, die solche Produkte sind, welche bei Raumtemperatur und unter Normaldruck gasförmig sind. Beispiele solcher Produkte sind insbesondere Aerosol-Treibgase, wie Halogenkohlenwasserstoffe, z.B. Dichlorodifluormethan. Liegt das erfindungsgemässe Unkrautbekämpfungsmittel in Form einer Druckgaspackung vor, so wird zweckmässigerweise zusätzlich zum Treibgas ein Lösungsmittel verwendet.

Die Tenside (Netz- und Emulgiermittel) können nichtionische Verbindungen sein, wie Kondensationsprodukte von Fettsäuren, Fettalkoholen oder fettsubstituierten Phenolen mit Aethylenoxid; Fettsäureester und -äther von Zuckern oder mehrwertigen Alkoholen; die Produkte, die aus Zuckern oder mehrwertigen Alkoholen durch Kondensation mit Aethylenoxid erhalten werden; Blockpolymere von Aethylenoxid und Propylenoxid; oder Alkyldimethylaminoxide.

Die Tenside können auch anionische Verbindungen darstellen, wie Seifen; Fettsulfatester, z.B. Dodecylnatriumsulfat, Octadecylnatriumsulfat und Cetylnatriumsulfat; Alkylsulfonate, Arylsulfonate und fettaromatische Sulfonate, wie Alkylbenzolsulfonate z.B. Calcium-dodecylbenzolsulfonat, und Butylnaphthalinsulfonate; und komplexere Fettsulfonate, z.B. die Amidkondensationsprodukte von Oelsäure und N-Methyltaurin und das Natriumsulfonat von Dioctylsuccinat.

Die Tenside können schliesslich kationische Verbindungen sein, wie Alkyldimethylbenzylammoniumchloride, Dialkyldimethylammoniumchloride, Alkyltrimethylammoniumchloride und äthoxylierte quaternäre Ammoniumchloride.

Als Dispergatoren (ohne Tensidwirkung) kommen im wesentlichen in Frage: Lignin, Natrium- und Ammoniumsalze von Ligninsulfonsäuren, Natriumsalze von Maleinsäureanhydrid-

Diisobutylen-Copolymeren, Natrium- und Ammoniumsalze von sulfonierten Polykondensationsprodukten aus Naphthalin und Formaldehyd, und Sulfitablaugen.

Als Dispergatoren, die sich insbesondere als Verdickungs- bzw. Antiabsetzmittel eignen, können z.B. Methylcellulose, Carboxymethylcellulose, Hydroxyäthylcellulose, Polyvinylalkohol, Alginate, Caseinate und Blutalbumin eingesetzt werden.

Beispiele von geeigneten Stabilisatoren sind säurebindende Mittel z.B. Epichlorhydrin, Phenylglycidäther und Soyaepoxide; Antioxidantien z.B. Gallussäureester und Butylhydroxytoluol; UV-Absorber z.B. substituierte Benzophenone, Diphenylacrylonitrilsäureester und Zimtsäureester; und Deaktivatoren z.B. Salze der Aethylendiaminotetraessigsäure und Polyglykole.

Die erfindungsgemässen Unkrautbekämpfungsmittel können zusätzlich zu den Verbindungen der Formel I Synergisten und andere Wirkstoffe, z.B. Insektizide, Akarizide, Bakterizide, anderweitige Herbizide, Fungizide, Pflanzenwachstumsregulatoren und Düngemittel, enthalten. Solche Kombinationsmittel eignen sich zur Verstärkung der Aktivität bzw. zur Verbreiterung des Wirkungsspektrums.

Die erfindungsgemässen Unkrautbekämpfungsmittel enthalten im allgemeinen zwischen 0,01 und 90 Gewichtsprozent, vorzugsweise zwischen 25 und 75 Gewichtsprozent einer bzw. mehrerer Verbindungen der Formel I als Wirkstoff(e). Sie können z.B. in einer Form vorliegen, die sich für die Lagerung und den Transport eignet. In solchen Formulierungen, z.B. emulgierbaren Konzentraten, ist die Wirkstoffkonzentration normalerweise im höherern Bereich, vorzugsweise zwischen 25 und 75 Gewichtsprozent, insbesondere zwischen 40 und 60 Gewichtsprozent. Diese Formulierungen können dann, z.B. mit gleichen oder verschiedenen inerten Stoffen, bis zu Wirkstoffkonzentrationen verdünnt werden, die sich

für den praktischen Gebrauch eignen, also vorzugsweise zwischen 0,05 und 5 Gewichtsprozent, insbesondere zwischen 0,1 und 1 Gewichtsprozent. Die Wirkstoffkonzentrationen können jedoch auch kleiner oder grösser sein.

Wie oben erwähnt, kann die Herstellung der erfindungsgemässen Unkrautbekämpfungsmittel in an sich bekannter Weise durchgeführt werden.

Zur Herstellung pulverförmiger Präparate kann der Wirkstoff, d.h. mindestens eine Verbindung der Formel I, mit festen Trägerstoff vermischt werden, z.B. durch Zusammenmahlen; oder man kann den festen Trägerstoff mit einer Lösung oder Suspension des Wirkstoffes imprägnieren und dann das Lösungs- bzw. Dispersionsmittel durch Abdunsten, Erhitzen oder Absaugen unter vermindertem Druck entfernen. Durch Zusatz von Tensiden bzw. Dispergatoren kann man solche pulverförmige Mittel mit Wasser leicht benetzbar machen, so dass sie in wässrige Suspensionen, die sich z.B. als Spritzmittel eignen, übergeführt werden können.

Die Verbindung der Formel I kann auch mit einem Tensid und einem festen Trägerstoff zur Bildung eines netzbaren Pulvers vermischt werden, welches in Wasser dispergierbar ist, oder sie kann mit einem festen vorgranulierten Trägerstoff zur Bildung eines granulatförmigen Produktes vermischt werden.

Wenn gewünscht, kann die Verbindung der Formel I in einem mit Wasser nicht mischbaren Lösungsmittel, wie beispielsweise einem hochsiedenden Kohlenwasserstoff, gelöst werden, das zweckmässigerweise gelöste Emulgiermittel enthält, so dass die Lösung bei Zugabe zu Wasser selbstemulgierend wirkt. Andernfalls kann der Wirkstoff mit einem Emulgiermittel vermischt und das Gemisch dann mit Wasser auf die gewünschte Konzentration verdünnt werden. Zudem kann der Wirkstoff in einem Lösungsmittel gelöst und danach mit einem Emulgiermittel gemischt werden. Ein solches Gemisch kann ebenfalls mit Wasser auf die gewünschte Konzentration verdünnt werden. Auf diese Weise erhält man emulgierbare Konzentrate bzw. gebrauchsfertige Emulsionen.

Das Unkrautbekämpfungsmittel kann auch in Form einer Druckgaspackung vorliegen, wobei zweckmässigerweise zusätzlich zum Treibmittel, welches geeigneterweise ein verflüssigtes polyhalogeniertes Alkan, wie Dichlordifluormethan, ist, ein Lösungsmittel verwendet wird.

Die Verwendung der erfindungsgemässen Unkrautbekämpfungsmittel kann nach üblichen Applikationsmethoden, wie Spritzen, Sprühen, Stäuben, Giessen oder Streuen, erfolgen. Das erfindungsgemässe Verfahren zur Bekämpfung von Unkräutern ist dadurch gekennzeichnet, dass man das gegen Unkräuter zu schützende Gut und/oder die Unkräuter mit einer erfindungsgemässen Verbindung bzw. einem erfindungsgemässen Unkrautbekämpfungsmittel behandelt.

Die nachfolgenden Beispiele dienen zur näheren Erläuterung der Erfindung.

I. Herstellung der Wirkstoffe der Formel I:

## Beispiel 1

Zu einer Lösung von 12,2 g Hydrazindithiocarbonsäure-methylester und 9,5 g Pyridin in 200 ml Benzol wird unter Rühren bei 40°C während 30 Minuten eine Lösung von 34,4 g D-2-[p-(α,α,α-Trifluor-p-tolyloxy)phenoxy]-propionsäure-chlorid in 100 ml Benzol zugetropft. Das Lösungsmittel wird dann im Vakuum abdestilliert, der Rückstand in 300 ml Aethylacetat gelöst und die Lösung mit Wasser ausgeschüttelt, über wasserfreiem Natriumsulfat getrocknet und zur Trockene eingedampft. Schliesslich wird der Rückstand in 50 ml Diäthyläther gelöst, die Lösung portionenweise mit 400 ml n-Hexan versetzt und der sich ergebende Niederschlag durch Filtrierung isoliert. Man erhält das Methyl-3-{D-2-[p-(α,α,α-trifluor-p-tolyloxy)phenoxy]-propionyl}-dithiocarbazat; Fp. 126-128°C; $[\alpha]_D^{22}$ +71,8° (c = 1,2% in $CHCl_3$).

In analoger Weise erhält man ausgehend von

Hydrazindithiocarbonsäure-methylester und D-2-[p-(p-Jodphenoxy)phenoxy]-propionsäurechlorid das Methyl-3-{D-2-[p-(p-jodphenoxy)phenoxy]-propionyl}-dithiocarbazat; Fp. 135-138°C; $[\alpha]_D^{22}$ +51,3° (c = 1,3% in $CHCl_3$).

Hydrazindithiocarbonsäure-benzylester und D-2-[p-(α,α,α-Trifluor-p-tolyloxy)phenoxy]-propionsäurechlorid das Benzyl-3-{D-2-[p-(α,α,α-trifluor-p-tolyloxy)phenoxy]-propionyl}-dithiocarbazat; Fp. 119-122°C; $[\alpha]_D^{22}$ +57,9° (c = 1,0% in $CHCl_3$).

## Beispiel 2

Eine Lösung von 3,4 g D-2-[p-(α,α,α-Trifluor-p-tolyloxy)phenoxy]-propionsäure-hydrazid in 20 ml Dichlormethan wird mit 2,0 g Essigsäureanhydrid versetzt. Die ausgefallenen Kristalle werden abgenutscht und aus Dichlormethan/n-Hexan umkristallisiert. Man erhält das 1-Acetyl-2-{D-2-[p-(α,α,α-trifluor-p-tolyloxy)phenoxy]-propionyl}-hydrazin; Fp. 178-180°C; $[\alpha]_D^{22}$ +19,2° (c = 1,2% in $C_2H_5OH$).

In analoger Weise erhält man ausgehend von

D-2-[p-(α,α,α-Trifluor-p-tolyloxy)phenoxy]-propionsäure-hydrazid und Chloressigsäureanhydrid das 1-Chloracetyl-2-{D-2-[p-(α,α,α-trifluor-p-tolyloxy)phenoxy]-propionyl}-hydrazin; Fp. 192-194°C; $[\alpha]_D^{22}$ +20,5° (c = 0,9% in $C_2H_5OH$).

## Beispiel 3

Eine Lösung von 3,4 g D-2-[p-(α,α,α-Trifluor-p-tolyloxy)phenoxy]-propionsäure-hydrazid und 1,7 g Benzoylchlorid in 40 ml Dichlormethan wird bei 10°C mit 1,6 g Pyridin versetzt und 15 Stunden bei 20°C stehengelassen. Danach wird das Reaktionsgemisch im Vakuum eingedampft, der feste Rückstand in 100 ml Diäthyläther gelöst und die Lösung mit Wasser ausgeschüttelt, über wasserfreiem Natriumsulfat getrocknet und zur Trockene eingedampft. Schliesslich wird der Rückstand aus Diäthyläther/n-Hexan kristallisiert. Man erhält das 1-Benzoyl-2-{D-2-[p-(α,α,α-trifluor-p-tolyloxy)phenoxy]-propionyl}-hydrazin; Fp. 158-160°C; $[\alpha]_D^{22}$ +14,8° (c = 0,7% in $CHCl_3$).

In analoger Weise erhält man ausgehend von

D-2-[p-(α,α,α-Trifluor-p-tolyloxy)phenoxy]-propionsäure-hydrazid und Methansulfochlorid das 1-Methansulfonyl-2-{D-2-[p-(α,α,α-trifluor-p-tolyloxy)phenoxy]-propionyl}-hydrazin; Fp. 125-128°C; $[\alpha]_D^{22}$ -20,0° (c = 1,4% in $C_2H_5OH$).

D-2-[p-(α,α,α-Trifluor-p-tolyloxy)phenoxy]-propionsäure-hydrazid und Toluol-4-sulfochlorid das 1-(Toluol-4-sulfonyl)-2-{D-2-[p-(α,α,α-trifluor-p-tolyloxy)phenoxy]-propionyl}-hydrazin; Fp. 165-168°C; $[\alpha]_D^{22}$ +55,6° (c = 1,0%

in $C_2H_5OH$).

D-2-[p-(α,α,α-Trifluor-p-tolyloxy)phenoxy]-propionsäure-hydrazid und Chlorameisensäure-äthylester das Aethyl-3-{D-2-[p-(α,α,α-trifluor-p-tolyloxy)phenoxy]-propionyl}-carbazat; Fp. 84-91°C; $[\alpha]_D^{22}$ -4,0° (c = 0,8% in $CHCl_3$).

D-2-[p-(p-Jodphenoxy)phenoxy]-propionsäure-hydrazid und Benzoylchlorid das 1-Benzoyl-2-{D-2-[p-(p-jodphenoxy)-phenoxy]-propionyl}-hydrazin; Fp. 182-184°C; $[\alpha]_D^{22}$ + 10,4° (c = 2,2% in $CHCl_3$).

D-2-[p-(p-Jodphenoxy)phenoxy]-propionsäure-hydrazid und Chlorameisensäure-äthylester das Aethyl-3-{D-2-[p-(p-jodphenoxy)phenoxy]-propionyl}-carbazat; Fp. 107-108°C; $[\alpha]_D^{22}$ -3,0° (c = 1,3% in $CHCl_3$).

D-2-[p-(o-Chlor-p-jodphenoxy)phenoxy]-propionsäure-hydrazid und Benzoylchlorid das 1-Benzoyl-2-{D-2-[p-(o-chlor-p-jodphenoxy)phenoxy]-propionyl}-hydrazin; Fp. 170-172°C; $[\alpha]_D^{22}$ + 10,7° (c = 1,6% in $CHCl_3$).

D-2-[p-(o-Chlor-p-jodphenoxy)phenoxy]-propionsäure-hydrazid und Chlorameisensäure-äthylester das Aethyl-3-{D-2-[p-(o-chlor-p-jodphenoxy)phenoxy]-propionyl}-carbazat; Fp. 115-118°C; $[\alpha]_D^{22}$ -2,2° (c = 1,5% in $CHCl_3$).

D-2-[p-(o-Chlor-α,α,α-trifluor-p-tolyloxy)phenoxy]-propionsäure-hydrazid und Benzoylchlorid das 1-Benzoyl-2-{D-2-[p-(o-chlor-α,α,α-trifluor-p-tolyloxy)phenoxy]-propionyl}-hydrazin; Fp. 139-144°C; $[\alpha]_D^{22}$ + 15,1° (c = 0,95% in $CHCl_3$).

D-2-[p-(o-Chlor-α,α,α-trifluor-p-tolyloxy)phenoxy]-propionsäure-hydrazid und Chlorameisensäure-äthylester das Aethyl-3-{D-2-[p-(o-chlor-α,α,α-trifluor-p-tolyloxy)phenoxy]-propionyl}-carbazat; $[\alpha]_D^{22}$ -3,3° (c = 0,94% in $CHCl_3$).

Beispiel 4

Eine Lösung von 4,3 g Methyl-3-{D-2-[p-(α,α,α-trifluor-p-tolyloxy)phenoxy]-propionyl}-dithiocarbazat und 1,5 g Natriumcarbonat in 125 ml Wasser und 75 ml Methanol wird bei 25°C mit 4,2 g Methyljodid versetzt und das Reaktionsgemisch 1 Stunde bei 35°C gerührt. Die ausgefallenen Kristalle werden abgenutscht, mit Wasser gewaschen und in Diäthyläther gelöst. Danach wird die Lösung über wasserfreiem Natriumsulfat getrocknet, auf 10 ml eingeengt und mit etwas n-Hexan zur Kristallisation gebracht. Man erhält auf diese Weise Kristalle von Dimethyl-N-{D-2-[p-(α,α,α-trifluor-p-tolyloxy)phenoxy]-propionamido}-dithioimidocarbonat; Fp. 97-99°C; $[\alpha]_D^{22}$ +63,2° (c = 1,6% in $CHCl_3$).

In analoger Weise erhält man ausgehend von

Methyl-3-{D-2-[p-(p-jodphenoxy)phenoxy]-propionyl}-dithiocarbazat und Propargylbromid unter Verwendung von wässrigem 1,2-Dimethoxyäthan als Lösungsmittel das Methyl-(2-propinyl)-N-{D-2-[p-(p-jodphenoxy)phenoxy]-propionamido}-dithioimidocarbonat; $n_D^{30}$ 1,6500; $[\alpha]_D^{22}$ +34,1° (c = 0,8% in $CHCl_3$).

Methyl-3-{D-2-[p-(α,α,α-trifluor-p-tolyloxy)phenoxy]-propionyl}-dithiocarbazat und Aethylbromid das Aethyl-methyl-N-{D-2-[p-(α,α,α-trifluor-p-tolyloxy)phenoxy]-propionamido}-dithioimidocarbonat; Fp. 73-76°C; $[\alpha]_D^{22}$ +55,0° (c = 1,2% in $CHCl_3$).

Methyl-3-{D-2-[p-(α,α,α-trifluor-p-tolyloxy)phenoxy]-propionyl}-dithiocarbazat und Propargylbromid das Methyl-(2-propinyl)-N-{D-2-[p-(α,α,α-trifluor-p-tolyloxy)phenoxy]-propionamido}-dithioimidocarbonat; $[\alpha]_D^{22}$ +54,4° (c = 0,8% in $CHCl_3$).

Methyl-3-{D-2-[p-(α,α,α-trifluor-p-tolyloxy)phenoxy]-propionyl}-dithiocarbazat und Isopropylbromid das Isopropyl-

methyl-N-{D-2-[p-(α,α,α-trifluor-p-tolyloxy)phenoxy]-propionamido}-dithioimidocarbonat; $n_D^{30}$ 1,5642; $[\alpha]_D^{22}$ +46,0° (c = 1,2% in $CHCl_3$).

Methyl-3-{D-2-[p-(α,α,α-trifluor-p-tolyloxy)phenoxy]-propionyl}-dithiocarbazat und Benzylbromid das Benzyl-methyl-N-{D-2-[p-(α,α,α-trifluor-p-tolyloxy)phenoxy]-propionamido}-dithioimidocarbonat; $n_D^{30}$ 1,5936; $[\alpha]_D^{22}$ +55,2° (c = 1,0% in $CHCl_3$).

Methyl-3-{D-2-[p-(α,α,α-trifluor-p-tolyloxy)phenoxy]-propionyl}-dithiocarbazat und Allylbromid das Allyl-methyl-N-{D-2-[p-(α,α,α-trifluor-p-tolyloxy)phenoxy]-propionamido}-dithioimidocarbonat; $n_D^{30}$ 1,5795; $[\alpha]_D^{22}$ +24,8° (c = 1,1% in $CHCl_3$).

Beispiel 5

Ein Gemisch von 34,0 g Methyl-D-2-[p-(α,α,α-trifluor-p-tolyloxy)phenoxy]-propionat und 25 g Hydrazinhydrat wird 4 Stunden bei 90°C gerührt.Nach Abkühlen wird der resultierende Kristallbrei mit 1 l Diäthyläther ausgeschüttelt und danach die Aetherphase mit Wasser gewaschen, über wasserfreiem Natriumsulfat getrocknet und zur Trockene eingedampft. Der Rückstand wird aus Diäthyläther/n-Pentan kristallisiert. Man erhält das D-2-[p-(α,α,α-Trifluor-p-tolyloxy)phenoxy]-propionsäure-hydrazid; Fp. 94-96°C; $[\alpha]_D^{20}$ -20,0° (c = 1,2% in $CHCl_3$).

Eine Lösung von 3,4 g D-2-[p-(α,α,α-Trifluor-p-tolyloxy)phenoxy]-propionsäure-hydrazid und 0,05 g p-Toluolsulfonsäure in 25 ml wasserfreiem Aceton wird 10 Stunden bei 25°C stehengelassen. Danach wird das Reaktionsgemisch zur Trockene eingedampft und der Rückstand an Kieselgel mit Dichlormethan/Aceton (4:1) chromatographisch gereinigt. Man erhält das 1-Isopropyliden-2-{D-2-[p-(α,α,α-trifluor-p-tolyloxy)phenoxy]-propionyl}-hydrazin; Fp. 93-95°C; $[\alpha]_D^{22}$ +50,7° (c = 0,8% in $CHCl_3$).

In analoger Weise erhält man ausgehend von

D-2-[p-(α,α,α-Trifluor-p-tolyloxy)phenoxy]-propionsäure-hydrazid und Diisopropylketon das 1-(1-Isopropyl-2-methylpropyliden)-2-{D-2-[p-(α,α,α-trifluor-p-tolyloxy)-phenoxy]-propionyl}-hydrazin; Fp. 99-102°C; $[\alpha]_D^{22}$ +15,52° (c = 1,2% in $CHCl_3$).

D-2-[p-(α,α,α-Trifluor-p-tolyloxy)phenoxy]-propionsäure-hydrazid und frisch destilliertem Trichloracetaldehyd unter Verwendung von Dichlormethan als Lösungsmittel das 1-(2,2,2-Trichloräthyliden)-2-{D-2-[p-(α,α,α-trifluor-p-tolyloxy)phenoxy]-propionyl}-hydrazin; Fp. 122-125°C; $[\alpha]_D^{22}$ -27,7° (c = 0,9% in $CHCl_3$).

D-2-[p-(α,α,α-Trifluor-p-tolyloxy)phenoxy]-propionsäure-hydrazid und Cyclohexan unter Verwendung von Dichlormethan als Lösungsmittel das 1-Cyclohexyliden-2-{D-2-[p-(α,α,α-trifluor-p-tolyloxy)phenoxy]-propionyl}-hydrazin; Fp. 122-124°C; $[\alpha]_D^{22}$ +57,7° (c = 0,6% in $CHCl_3$).

D-2-[p-(α,α,α-Trifluor-p-tolyloxy)phenoxy]-propionsäure-hydrazid und Benzaldehyd unter Verwendung von Dichlormethan als Lösungsmittel das 1-Benzyliden-2-{D-2-[p-(α,α,α-trifluor-p-tolyloxy)phenoxy]-propionyl}-hydrazin; Fp. 174-177°C; $[\alpha]_D^{22}$ -57,8° (c = 1,0% in $CHCl_3$).

D-2-[p-(α,α,α-Trifluor-p-tolyloxy)phenoxy]-propionsäure-hydrazid und Acetophenon unter Verwendung von Dichlormethan als Lösungsmittel das 1-(α-Methylbenzyliden)-2-{D-2-[p-(α,α,α-trifluor-p-tolyloxy)phenoxy]-propionyl}-hydrazin; Fp. 99-101°C; $[\alpha]_D^{22}$ -79,3° (c = 0,6% in $CHCl_3$).

D-2-[p-(o-Chlor-p-jodphenoxy)phenoxy]-propionsäure-hydrazid und Aceton das 1-Isopropyliden-2-{D-2-[p-(o-chlor-p-jodphenoxy)phenoxy]-propionyl}-hydrazin; $[\alpha]_D^{22}$ +50,9° (c = 1,1% in $CHCl_3$).

D-2-[p-(p-Jodphenoxy)phenoxy]-propionsäure-hydrazid und Aceton das 1-Isopropyliden-2-{D-2-[p-(p-jodphenoxy)-phenoxy]-propionyl}-hydrazin; $[\alpha]_D^{22}$ + 48,3° (c = 1,1% in $CHCl_3$).

D-2-[p-(o-Chlor-α,α,α-trifluor-p-tolyloxy)phenoxy]-propionsäure-hydrazid und Aceton das 1-Isopropyliden-2-{D-2-[p-(o-chlor-α,α,α-trifluor-p-tolyloxy)phenoxy]-propionyl}-hydrazin; Fp. 115-119°C; $[\alpha]_D^{22}$ + 50,04° (c = 1,23% in $CHCl_3$).

Die in obigen Verfahren verwendeten Ausgangsmaterialien D-2-[p-(o-Chlor-p-jodphenoxy)phenoxy]-propionsäure-hydrazid [Fp. 98-102°; $[\alpha]_D^{20}$ -13,0° (c = 1,2% in $CHCl_3$)], D-2-[p-(p-Jodphenoxy)phenoxy]-propionsäure-hydrazid und D-2-[p-(o-Chlor-α,α,α-trifluor-p-tolyloxy)phenoxy]-propionsäure-hydrazid [Fp. 105-108°C; $[\alpha]_D^{22}$ -15,3° (c = 0,84% in $CHCl_3$)] werden in analoger Weise zum oben beschriebenen Verfahren zur Herstellung von D-2-[p-(α,α,α-Trifluor-p-tolyloxy)phenoxy]-propionsäure-hydrazid hergestellt, und zwar aus Methyl-D-2-[p-(o-chlor-p-jodphenoxy)phenoxy]-propionat bzw. Methyl-D-2-[p-(p-jodphenoxy)phenoxy]-propionat bzw. Methyl-D-2-[p-(o-chlor-α,α,α-trifluor-p-tolyloxy)phenoxy]-propionat und Hydrazinhydrat.

## II. Formulierungsbeispiele:

### Beispiel 6

Zur Herstellung eines emulgierbaren Konzentrates werden die nachstehend aufgeführten Bestandteile miteinander vermischt:

| | |
|---|---|
| Verbindung der Formel I | 500 g |
| Gemisch aus Kondensationsprodukten eines Alkylphenols und Aethylenoxid und Dodecylbenzolsulfon-saurem Calcium | 100 g |
| Epoxydiertes Soyaöl mit einem Oxiransauerstoff- | |

| | |
|---|---|
| gehalt von ca.6% | 25 g |
| Butyliertes Hydroxytoluol | 10 g |

Diese Mischung wird mit Xylol auf 1 Liter aufgefüllt.

Das so erhaltene Konzentrat emulgiert spontan in Wasser. Die sich ergebende Emulsion eignet sich als gebrauchsfertige Spritzbrühe.

## Beispiel 7

Zur Herstellung eines emulgierbaren Konzentrates werden die nachstehend aufgeführten Bestandteile miteinander vermischt:

| | |
|---|---|
| Verbindung der Formel I | 250 g/l |
| N-Methyl-2-pyrrolidon | 300 g/l |
| Emulgator A[1)] | 100 g/l |
| Emulgator B[2)] | 25 g/l |
| Lösungsmittelgemisch aus Alkylbenzolen | auf 1000 ml |

[1)]Emulgator A: Emulgator bestehend aus 60 Teilen eines Blockpolymerisates aus Aethylenoxid und Propylenoxid, 20 Teilen des Calciumsalzes einer verzweigtkettigen Dodecylbenzolsulfonsäure und 20 Teilen eines Lösungsmittelgemisches aus Isobutanol und $C_{10}$-Alkylbenzolen.

[2)]Emulgator B: Gemisch aus 70 Teilen des Calciumsalzes einer verzweigtkettigen Dodecylbenzolsulfonsäure und 30 Teilen eines Lösungsmittelgemisches aus Isobutanol und $C_{10}$-Alkylbenzolen.

Das so erhaltene Konzentrat emulgiert spontan in Wasser. Die sich ergebende Emulsion eignet sich als gebrauchsfertige Spritzbrühe.

## Patentansprüche

1. Verbindungen der allgemeinen Formel

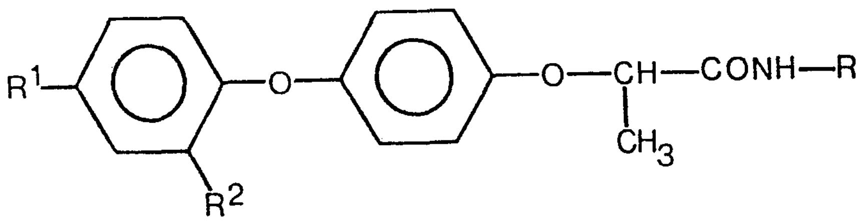 I

worin $R^1$ Chlor, Jod oder Trifluormethyl,
$R^2$ Wasserstoff oder Chlor
und R eine der folgenden Gruppen (a) - (e) bedeuten:

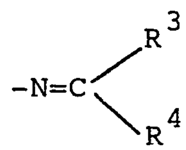 (a)

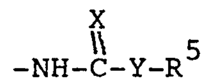 (b)

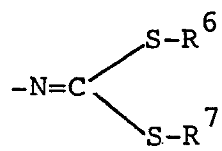 (c)

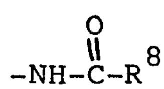 (d)

$-NH-SO_2-R^9$ (e)

wobei in den obigen Formeln der Gruppen (a) - (e) $R^3$ und $R^4$ Wasserstoff; $C_{1-4}$-Alkyl; $C_{1-4}$-Halogenalkyl; Phenyl, gegebenenfalls substituiert mit Chlor, Methyl, Methoxy und/oder Nitro; oder Benzyl, gegebenenfalls substituiert im Arylkern mit Chlor, Methyl, Methoxy und/oder Nitro, bedeuten oder zusammen mit dem Kohlenstoffatom, an das sie geknüpft sind, einen $C_{5-7}$-Cycloalkanring bilden,
$R^5$ $C_{1-4}$-Alkyl, $C_{2-4}$-Alkenyl, $C_{2-4}$-Alkinyl, Phenyl oder Benzyl bedeutet,
X und Y Sauerstoff oder Schwefel bedeuten,

$R^6$ und $R^7$ $C_{1-4}$-Alkyl, $C_{2-4}$-Alkenyl, $C_{2-4}$-Alkinyl, Phenyl oder Benzyl bedeuten oder zusammen eine $C_{2-4}$-Alkylengruppe bilden, mit der Massgabe, dass $R^6$ und $R^7$ nicht gleichzeitig Phenyl bedeuten,

$R^8$ Wasserstoff; $C_{1-4}$-Alkyl; $C_{1-4}$-Halogenalkyl; $C_{2-4}$-Alkenyl; oder Phenyl, gegebenenfalls substituiert mit Chlor, Methyl, Methoxy und/oder Nitro, bedeutet

und $R^9$ $C_{1-4}$-Alkyl; oder Phenyl, gegebenenfalls substituiert mit Chlor, Methyl, Methoxy und/oder Nitro, bedeutet.

2. Verbindungen nach Anspruch 1, worin $R^1$ Jod oder Trifluormethyl bedeutet.

3. Verbindungen nach Anspruch 2, worin $R^1$ Jod und $R^2$ Chlor bedeuten.

4. Verbindungen nach Anspruch 2, worin $R^1$ Trifluormethyl und $R^2$ Chlor bedeuten.

5. Die D-Formen der Verbindungen nach einem der Ansprüche 1 bis 4.

6. I-Isopropyliden-2-{D-2-[p-(α,α,α-trifluor-p-tolyloxy)phenoxy]-propionyl}-hydrazin.

7. 1-Isopropyliden-2-{D-2-[p-(o-chlor-p-jodphenoxy)-phenoxy]-propionyl}-hydrazin.

8. 1-Isopropyliden-2-{D-2-[p-(o-chlor-α,α,α-trifluor-p-tolyloxy)phenoxy]-propionyl}-hydrazin.

9. Aethyl-3-{D-2-[p-(o-chlor-α,α,α-trifluor-p-tolyloxy)phenoxy]-propionyl}-carbazat.

10. Eine Verbindung nach Anspruch 1, ausgewählt aus:

Methyl-3-{D-2-[p-(α,α,α-trifluor-p-tolyloxy)phenoxy]-propionyl}-dithiocarbazat,

Methyl-3-{D-2-[p-(p-jodphenoxy)phenoxy]-propionyl}-dithiocarbazat,

Benzyl-3-{D-2-[p-(α,α,α-trifluor-p-tolyloxy)phenoxy]-propionyl}-dithiocarbazat,

1-Acetyl-2-{D-2-[p-(α,α,α-trifluor-p-tolyloxy)phenoxy]-propionyl}-hydrazin,

1-Chloracetyl-2-{D-2-[p-(α,α,α-trifluor-p-tolyloxy)-phenoxy]-propionyl}-hydrazin,

1-Benzoyl-2-{D-2-[p-(α,α,α-trifluor-p-tolyloxy)phenoxy]-propionyl}-hydrazin,

1-Methansulfonyl-2-{D-2-[p-(α,α,α-trifluor-p-tolyloxy)-phenoxy]-propionyl}-hydrazin,

1-(Toluol-4-sulfonyl)-2-{D-2-[p-(α,α,α-trifluor-p-tolyloxy)phenoxy]-propionyl}-hydrazin,

Aethyl-3-{D-2-[p-(α,α,α-trifluor-p-tolyloxy)phenoxy]-propionyl}-hydrazin,

Dimethyl-N-{D-2-[p-(α,α,α-trifluor-p-tolyloxy)phenoxy]-propionamido}-dithioimidocarbonat,

Methyl-(2-propinyl)-N-{D-2-[p-(p-jodphenoxy)phenoxy]-propionamido}-dithioimidocarbonat,

Aethyl-methyl-N-{D-2-[p-(α,α,α-trifluor-p-tolyloxy)-phenoxy]-propionamido}-dithioimidocarbonat,

Methyl-(2-propinyl)-N-{D-2-[p-(α,α,α-trifluor-p-tolyloxy)phenoxy]-propionamido}-dithioimidocarbonat,

Isopropyl-methyl-N-{D-2-[p-(α,α,α-trifluor-p-tolyloxy)phenoxy]-propionamido}-dithioimidocarbonat,

Benzyl-methyl-N-{D-2-[p-(α,α,α-trifluor-p-tolyloxy)-phenoxy]-propionamido}-dithioimidocarbonat,

Allyl-methyl-N-{D-2-[p-(α,α,α-trifluor-p-tolyloxy)-phenoxy]-propionamido}-dithioimidocarbonat,

1-(1-Isopropyl-2-methylpropyliden)-2-{D-2-[p-(α,α,α-trifluor-p-tolyloxy)phenoxy]-propionyl}-hydrazin,

1-(2,2,2-Trichloräthyliden)-2-{D-2-[p-(α,α,α-trifluor-p-tolyloxy)phenoxy]-propionyl}-hydrazin,

1-Cyclohexyliden)-2-{D-2-[p-(α,α,α-trifluor-p-tolyloxy)phenoxy]-propionyl}-hydrazin,

1-Benzyliden-2-{D-2-[p-(α,α,α-trifluor-p-tolyloxy)-phenoxy]-propionyl}-hydrazin und

1-(α-Methylbenzyliden)-2-{D-2-[p-(α,α,α-trifluor-p-tolyloxy)phenoxy]-propionyl}-hydrazin.

11. Eine Verbindung nach Anspruch 1, ausgewählt aus:

1-Benzoyl-2-{D-2-[p-(p-jodphenoxy)phenoxy]-propionyl}-hydrazin,

Aethyl-3-{D-2-[p-(p-jodphenoxy)phenoxy]-propionyl}-carbazat,

1-Benzoyl-2-{D-2-[p-(o-chlor-p-jodphenoxy)phenoxy]-propionyl}-hydrazin,

Aethyl-3-{D-2-[p-(o-chlor-p-jodphenoxy)phenoxy]-propionyl}-carbazat,

1-Benzoyl-2-{D-2-[p-(o-chlor-α,α,α-trifluor-p-tolyloxy)-phenoxy]-propionyl}-hydrazin und

1-Isopropyliden-2-{D-2-[p-(p-jodphenoxy)phenoxy]-propionyl}-hydrazin.

12. Eine Verbindung nach einem der Ansprüche 1 bis 3, 6 und 10 als Unkrautbekämpfungsmittel.

13. Eine Verbindung nach einem der Ansprüche 4, 5, 7 bis 9 und 11 als Unkrautbekämpfungsmittel.

14. Unkrautbekämpfungsmittel, dadurch gekennzeichnet, dass es eine wirksame Menge mindestens einer Verbindung der allgemeinen Formel

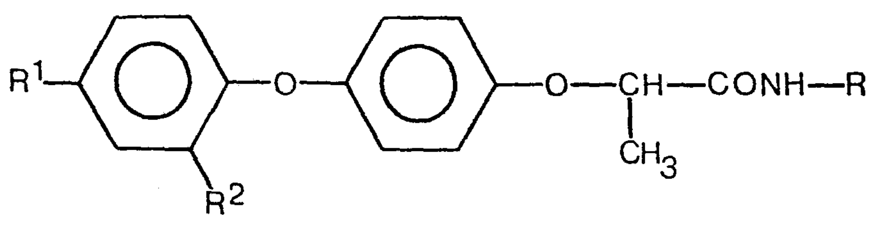 I

worin $R^1$ Chlor, Jod oder Trifluormethyl,
$R^2$ Wasserstoff oder Chlor
und R eine der folgenden Gruppen (a) - (e) bedeuten:

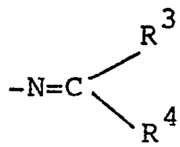
(a)

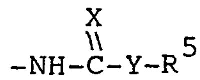
(b)

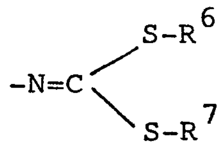
(c)

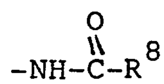
(d)

$-NH-SO_2-R^9$ (e)

wobei in den obigen Formeln der Gruppen (a) - (e) $R^3$ und $R^4$ Wasserstoff; $C_{1-4}$-Alkyl; $C_{1-4}$-Halogenalkyl; Phenyl, gegebenenfalls substituiert mit Chlor, Methyl, Methoxy und/oder Nitro; oder Benzyl, gegebenenfalls substituiert im Arylkern mit Chlor, Methyl, Methoxy und/oder Nitro, bedeuten oder zusammen mit dem Kohlenstoffatom, an das sie geknüpft sind, einen $C_{5-7}$-Cycloalkanring bilden,
$R^5$ $C_{1-4}$-Alkyl, $C_{2-4}$-Alkenyl, $C_{2-4}$-Alkinyl, Phenyl oder Benzyl bedeutet,
X und Y Sauerstoff oder Schwefel bedeuten,
$R^6$ und $R^7$ $C_{1-4}$-Alkyl, $C_{2-4}$-Alkenyl, $C_{2-4}$-Alkinyl, Phenyl oder Benzyl bedeuten oder zusammen eine $C_{2-4}$-Alkylengruppe bilden, mit der Massgabe, dass $R^6$ und $R^7$ nicht gleichzeitig Phenyl bedeuten,
$R^8$ Wasserstoff; $C_{1-4}$-Alkyl; $C_{1-4}$-Halogenalkyl; $C_{2-4}$-Alkenyl; oder Phenyl, gegebenenfalls substituiert mit Chlor, Methyl, Methoxy und/oder

Nitro, bedeutet

und $R^9$ $C_{1-4}$-Alkyl; oder Phenyl, gegebenenfalls substituiert mit Chlor, Methyl, Methoxy und/oder Nitro, bedeutet,

sowie Formulierungshilfsstoffe enthält.

15. Unkrautbekämpfungsmittel nach Anspruch 14, dadurch gekennzeichnet, dass es eine wirksame Menge von 1-Isopropyliden-2-{D-2-p-(α,α,α-trifluor-p-tolyloxy)-phenoxy]-propionyl}-hydrazin enthält.

16. Unkrautbekämpfungsmittel nach Anspruch 14, dadurch gekennzeichnet, dass es eine wirksame Menge einer der in den Ansprüchen 7 bis 9 genannten Verbindungen enthält.

17. Verfahren zur Herstellung von Verbindungen der allgemeinen Formel

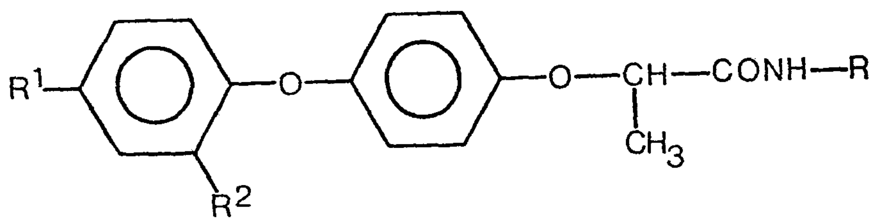

I

worin $R^1$ Chlor, Jod oder Trifluormethyl,

$R^2$ Wasserstoff oder Chlor

und R eine der folgenden Gruppen (a) - (e) bedeuten:

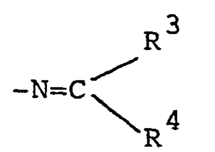

(a)

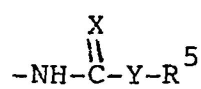

(b)

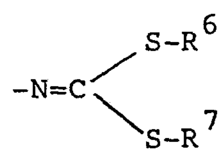

(c)

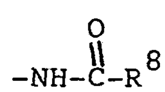

(d)

$-NH-SO_2-R^9$ (e)

wobei in den obigen Formeln der Gruppen (a) - (e) $R^3$ und $R^4$ Wasserstoff; $C_{1-4}$-Alkyl; $C_{1-4}$-Halogenalkyl; Phenyl, gegebenenfalls substituiert mit Chlor, Methyl, Methoxy und/oder Nitro; oder Benzyl, gegebenenfalls substituiert im Arylkern mit Chlor, Methyl, Methoxy und/oder Nitro, bedeuten oder zusammen mit dem Kohlenstoffatom, an das sie geknüpft sind, einen $C_{5-7}$-Cycloalkanring bilden,

$R^5$ $C_{1-4}$-Alkyl, $C_{2-4}$-Alkenyl, $C_{2-4}$-Alkinyl, Phenyl oder Benzyl bedeutet,

X und Y Sauerstoff oder Schwefel bedeuten,

$R^6$ und $R^7$ $C_{1-4}$-Alkyl, $C_{2-4}$-Alkenyl, $C_{2-4}$-Alkinyl, Phenyl oder Benzyl bedeuten oder zusammen eine $C_{2-4}$-Alkylengruppe bilden, mit der Massgabe, dass $R^6$ und $R^7$ nicht gleichzeitig Phenyl bedeuten,

$R^8$ Wasserstoff; $C_{1-4}$-Alkyl; $C_{1-4}$-Halogenalkyl; $C_{2-4}$-Alkenyl; oder Phenyl, gegebenenfalls substituiert mit Chlor, Methyl, Methoxy und/oder Nitro, bedeutet

und $R^9$ $C_{1-4}$-Alkyl; oder Phenyl, gegebenenfalls substituiert mit Chlor, Methyl, Methoxy und/oder Nitro, bedeutet,

dadurch gekennzeichnet, dass man

a) eine Säure der Formel

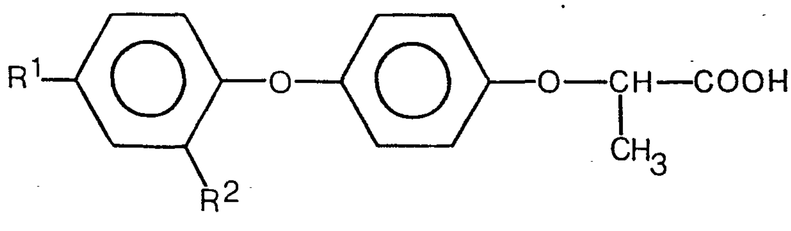

II

worin $R^1$ und $R^2$ die oben angegebenen Bedeutungen besitzen,

oder ein reaktionsfähiges Derivat davon, mit einem Hydrazinderivat der Formel

$H_2N-R$ III

worin R die oben angegebenen Bedeutungen besitzt, umsetzt,

b) ein Hydrazid der Formel

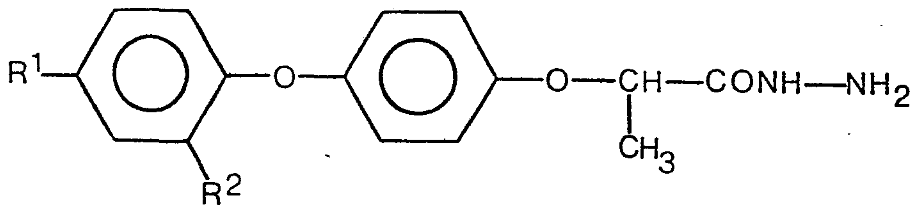

IV

worin $R^1$ und $R^2$ die oben angegebenen Bedeutungen besitzen,

mit einer Säure der Formel

$$HO-\overset{X}{\overset{\|}{C}}-Y-R^5 \qquad Va$$

$$HO-CO-R^8 \qquad Vb$$

$$HO-SO_2-R^9 \qquad Vc$$

worin $R^5$, X, Y, $R^8$ und $R^9$ die oben angegebenen Bedeutungen besitzen,

oder einem reaktionsfähigen Derivat davon, umsetzt,

c) eine Dithiocarbazinsäure der Formel

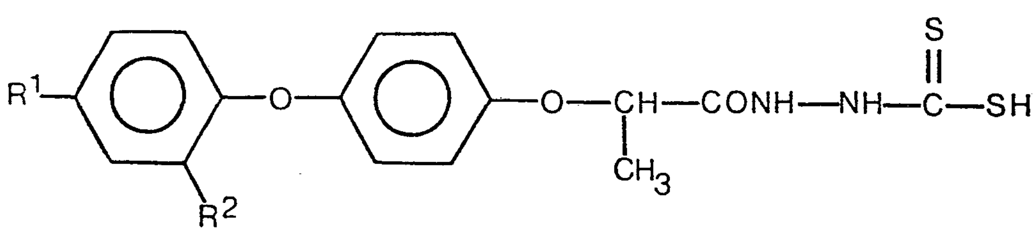

VI

worin $R^1$ und $R^2$ die oben angegebenen Bedeutungen besitzen,

oder ein Alkalimetall- oder Erdalkalimetallsalz davon, mit einer Verbindung der Formel

$$R^5-Z^1 \qquad VII$$

worin $R^5$ die oben angegebenen Bedeutungen besitzt und $Z^1$ eine Abgangsgruppe bedeutet,

umsetzt,

d) ein Dithiocarbazat der Formel

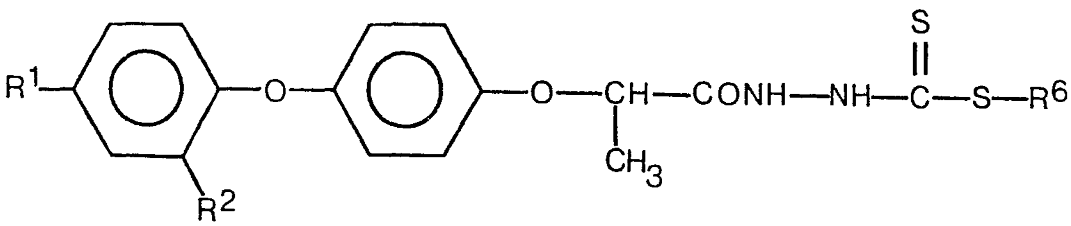

VIII

worin $R^1$, $R^2$ und $R^6$ die oben angegebenen Bedeutungen besitzen,

oder ein Alkalimetall- oder Erdalkalimetallsalz davon, mit einer Verbindung der Formel

$R^7Z^1$ IX

worin $R^7$ und $Z^1$ die oben angegebenen Bedeutungen besitzen,

umsetzt,

e) ein Hydrazid der Formel IV, wie oben definiert, mit einem Aldehyd bzw. Keton der Formel

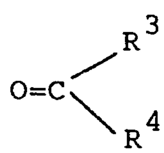

X

worin $R^3$ und $R^4$ die oben angegebenen Bedeutungen besitzen,

umsetzt, oder

f) ein Phenol der Formel

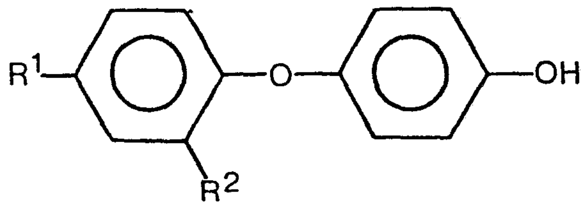 XI

worin $R^1$ und $R^2$ die oben angegebenen Bedeutungen besitzen,

oder ein Alkalimetallsalz davon, mit einem Hydrazinderivat der Formel

$Z^2$-CH-CONH-R XII
|
$CH_3$

worin R die oben angegebenen Bedeutungen besitzt, und $Z^2$ eine Abgangsgruppe bedeutet,

umsetzt.

18. Verfahren zur Bekämpfung von Unkräutern, dadurch gekennzeichnet, dass man das gegen Unkräuter zu schützende Gut und/oder die Unkräuter mit einer wirksamen Menge einer der in den Ansprüchen 1 bis 11 genannten Verbindungen bzw. eines der in den Ansprüchen 14 bis 16 genannten Mittel behandelt.

19. Verwendung einer der in den Ansprüchen 1 bis 11 genannten Verbindungen bzw. eines der in den Ansprüchen 14 bis 16 genannten Mittel zur Bekämpfung von Unkräutern.

***

Patentansprüche
OESTERREICH.

1. Unkrautbekämpfungsmittel, dadurch gekennzeichnet, dass es eine wirksame Menge mindestens einer Verbindung der allgemeinen Formel

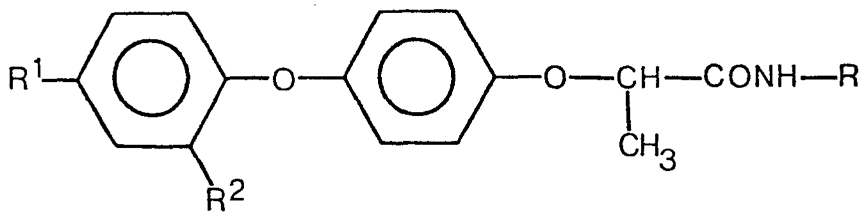 I

worin $R^1$ Chlor, Jod oder Trifluormethyl,
$R^2$ Wasserstoff oder Chlor
und R eine der folgenden Gruppen (a) - (e) bedeuten:

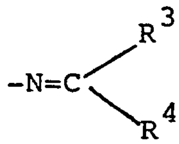 (a)

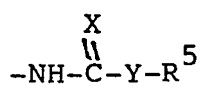 (b)

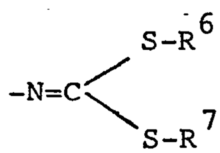 (c)

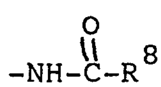 (d)

$-NH-SO_2-R^9$ (e)

wobei in den obigen Formeln der Gruppen (a) - (e) $R^3$ und $R^4$ Wasserstoff; $C_{1-4}$-Alkyl; $C_{1-4}$-Halogenalkyl; Phenyl, gegebenenfalls substituiert mit Chlor, Methyl, Methoxy und/oder Nitro; oder Benzyl, gegebenenfalls substituiert im Arylkern mit Chlor, Methyl, Methoxy und/oder Nitro, bedeuten oder zusammen mit dem Kohlenstoffatom, an das sie geknüpft sind, einen $C_{5-7}$-Cycloalkanring bilden,

$R^5$ $C_{1-4}$-Alkyl, $C_{2-4}$-Alkenyl, $C_{2-4}$-Alkinyl, Phenyl oder Benzyl bedeutet,
X und Y Sauerstoff oder Schwefel bedeuten,
$R^6$ und $R^7$ $C_{1-4}$-Alkyl, $C_{2-4}$-Alkenyl, $C_{2-4}$-Alkinyl, Phenyl oder Benzyl bedeuten oder zusammen eine $C_{2-4}$-Alkylengruppe bilden, mit der Massgabe, dass $R^6$ und $R^7$ nicht gleichzeitig Phenyl bedeuten,
$R^8$ Wasserstoff; $C_{1-4}$-Alkyl; $C_{1-4}$-Halogenalkyl; $C_{2-4}$-Alkenyl; oder Phenyl, gegebenenfalls substituiert mit Chlor, Methyl, Methoxy und/oder Nitro, bedeutet
und $R^9$ $C_{1-4}$-Alkyl; oder Phenyl, gegebenenfalls substituiert mit Chlor, Methyl, Methoxy und/oder Nitro, bedeutet,
sowie Formulierungshilfsstoffe enthält.

2. Unkrautbekämpfungsmittel nach Anspruch 1, worin $R^1$ in der Formel I Jod oder Trifluormethyl bedeutet.

3. Unkrautbekämpfungsmittel nach Anspruch 2, worin $R^1$ Jod und $R^2$ Chlor bedeuten.

4. Unkrautbekämpfungsmittel nach Anspruch 2, worin $R^1$ Trifluormethyl und $R^2$ Chlor bedeuten.

5. Unkrautbekämpfungsmittel nach einem der Ansprüche 1 bis 4, worin als Verbindungen der Formel I deren D-Formen verwendet werden.

6. Unkrautbekämpfungsmittel nach Anspruch 1, dadurch gekennzeichnet, dass es eine wirksame Menge von I-Isopropyliden-2-{D-2-[p-(α,α,α-trifluor-p-tolyloxy)phenoxy]-propionyl}-hydrazin enthält.

7. Unkrautbekämpfungsmittel nach Anspruch 1, dadurch gekennzeichnet, dass es eine wirksame Menge von 1-Isopropyliden-2-{D-2-[p-(o-chlor-p-jodphenoxy)phenoxy]-pro-

pionyl}-hydrazin enthält.

8. Unkrautbekämpfungsmittel nach Anspruch 1, dadurch gekennzeichnet, dass es eine wirksame Menge von 1-Isopropyliden-2-{D-2-[p-(o-chlor-α,α,α-trifluor-p-tolyloxy)-phenoxy]-propionyl}-hydrazin enthält.

9. Unkrautbekämpfungsmittel nach Anspruch 1, dadurch gekennzeichnet, dass es eine wirksame Menge von Aethyl-3-{D-2-[p-(o-chlor-α,α,α-trifluor-p-tolyloxy)phenoxy]-propionyl}-carbazat enthält.

10. Unkrautbekämpfungsmittel nach Anspruch 1, dadurch gekennzeichnet, dass es eine wirksame Menge mindestens einer Verbindung ausgewählt aus:

Methyl-3-{D-2-[p-(α,α,α-trifluor-p-tolyloxy)phenoxy]-propionyl}-dithiocarbazat,

Methyl-3-{D-2-[p-(p-jodphenoxy)phenoxy]-propionyl}-dithiocarbazat,

Benzyl-3-{D-2-[p-(α,α,α-trifluor-p-tolyloxy)phenoxy]-propionyl}-dithiocarbazat,

1-Acetyl-2-{D-2-[p-(α,α,α-trifluor-p-tolyloxy)phenoxy]-propionyl}-hydrazin,

1-Chloracetyl-2-{D-2-[p-(α,α,α-trifluor-p-tolyloxy)-phenoxy]-propionyl}-hydrazin,

1-Benzoyl-2-{D-2-[p-(α,α,α-trifluor-p-tolyloxy)phenoxy]-propionyl}-hydrazin,

1-Methansulfonyl-2-{D-2-[p-(α,α,α-trifluor-p-tolyloxy)-phenoxy]-propionyl}-hydrazin,

1-(Toluol-4-sulfonyl)-2-{D-2-[p-(α,α,α-trifluor-p-tolyloxy)phenoxy]-propionyl}-hydrazin,

Aethyl-3-{D-2-[p-(α,α,α-trifluor-p-tolyloxy)phenoxy]-propionyl}-hydrazin,

Dimethyl-N-{D-2-[p-(α,α,α-trifluor-p-tolyloxy)phenoxy]-propionamido}-dithioimidocarbonat,

Methyl-(2-propinyl)-N-{D-2-[p-(p-jodphenoxy)phenoxy]-propionamido}-dithioimidocarbonat,

Aethyl-methyl-N-{D-2-[p-(α,α,α-trifluor-p-tolyloxy)-phenoxy]-propionamido}-dithioimidocarbonat,

Methyl-(2-propinyl)-N-{D-2-[p-(α,α,α-trifluor-p-tolyloxy)phenoxy]-propionamido}-dithioimidocarbonat,

Isopropyl-methyl-N-{D-2-[p-(α,α,α-trifluor-p-tolyloxy)phenoxy]-propionamido}-dithioimidocarbonat,

Benzyl-methyl-N-{D-2-[p-(α,α,α-trifluor-p-tolyloxy)-phenoxy]-propionamido}-dithioimidocarbonat,

Allyl-methyl-N-{D-2-[p-(α,α,α-trifluor-p-tolyloxy)-phenoxy]-propionamido}-dithioimidocarbonat,

1-(1-Isopropyl-2-methylpropyliden)-2-{D-2-[p-(α,α,α-trifluor-p-tolyloxy)phenoxy]-propionyl}-hydrazin,

1-(2,2,2-Trichloräthyliden)-2-{D-2-[p-(α,α,α-trifluor-p-tolyloxy)phenoxy]-propionyl}-hydrazin,

1-Cyclohexyliden)-2-{D-2-[p-(α,α,α-trifluor-p-tolyloxy)phenoxy]-propionyl}-hydrazin,

1-Benzyliden-2-{D-2-[p-(α,α,α-trifluor-p-tolyloxy)-phenoxy]-propionyl}-hydrazin und

1-(α-Methylbenzyliden-2-{D-2-[p-(α,α,α-trifluor-p-tolyloxy)phenoxy]-propionyl}-hydrazin enthält.

11. Unkrautbekämpfungsmittel nach Anspruch 1, dadurch gekennzeichnet, dass es eine wirksame Menge mindestens einer Verbindung ausgewählt aus:

1-Benzoyl-2-{D-2-[p-(p-jodphenoxy)phenoxy]-propionyl}-hydrazin,

Aethyl-3-{D-2-[p-(p-jodphenoxy)phenoxy]-propionyl}-carbazat,

1-Benzoyl-2-{D-2-[p-(o-chlor-p-jodphenoxy)phenoxy]-propionyl}-hydrazin,

Aethyl-3-{D-2-[p-(o-chlor-p-jodphenoxy)phenoxy]-propionyl}-carbazat,

1-Benzoyl-2-{D-2-[p-(o-chlor-α,α,α-trifluor-p-tolyloxy)-phenoxy]-propionyl}-hydrazin und

1-Isopropyliden-2-{D-2-[p-(p-jodphenoxy)phenoxy]-propionyl}-hydrazin enthält.

12. Verfahren zur Herstellung von Verbindungen der allgemeinen Formel

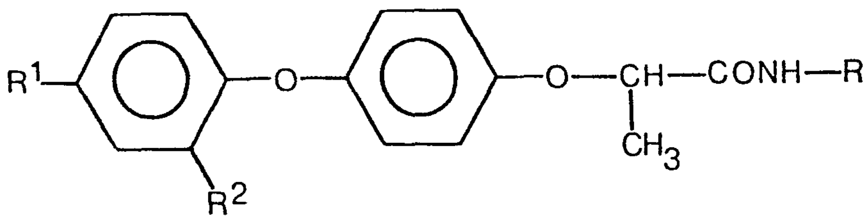

I

worin $R^1$ Chlor, Jod oder Trifluormethyl,
$R^2$ Wasserstoff oder Chlor
und R eine der folgenden Gruppen (a) - (e) bedeuten:

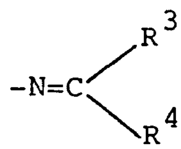

(a)

$$-NH-\overset{X}{\overset{\|}{C}}-Y-R^5$$ (b)

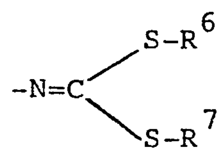

(c)

$$-NH-\overset{O}{\overset{\|}{C}}-R^8$$ (d)

$$-NH-SO_2-R^9$$ (e)

wobei in den obigen Formeln der Gruppen (a) - (e) $R^3$ und $R^4$ Wasserstoff; $C_{1-4}$-Alkyl; $C_{1-4}$-Halogenalkyl; Phenyl, gegebenenfalls substituiert mit Chlor, Methyl, Methoxy und/oder Nitro; oder Benzyl, gegebenenfalls substituiert im Arylkern mit Chlor, Methyl, Methoxy und/oder Nitro, bedeuten oder zusammen mit dem Kohlenstoffatom, an das sie geknüpft sind, einen $C_{5-7}$-Cycloalkanring bilden,
$R^5$ $C_{1-4}$-Alkyl, $C_{2-4}$-Alkenyl, $C_{2-4}$-Alkinyl, Phenyl oder Benzyl bedeutet,
X und Y Sauerstoff oder Schwefel bedeuten,
$R^6$ und $R^7$ $C_{1-4}$-Alkyl, $C_{2-4}$-Alkenyl, $C_{2-4}$-Alkinyl, Phenyl oder Benzyl bedeuten oder zusammen eine [illegible]engruppe bilden, mit der Massgabe,

dass $R^6$ und $R^7$ nicht gleichzeitig Phenyl bedeuten,

$R^8$ Wasserstoff; $C_{1-4}$-Alkyl; $C_{1-4}$-Halogenalkyl; $C_{2-4}$-Alkenyl; oder Phenyl, gegebenenfalls substituiert mit Chlor, Methyl, Methoxy und/oder Nitro, bedeutet

und $R^9$ $C_{1-4}$-Alkyl; oder Phenyl, gegebenenfalls substituiert mit Chlor, Methyl, Methoxy und/oder Nitro, bedeutet,

dadurch gekennzeichnet, dass man

a) eine Säure der Formel

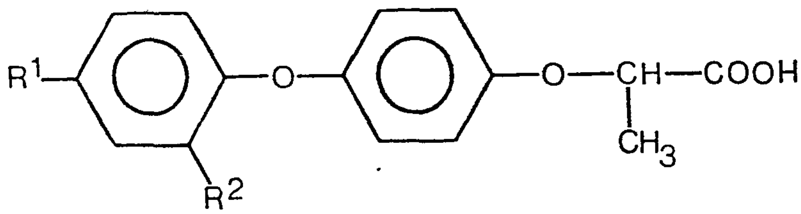

II

worin $R^1$ und $R^2$ die oben angegebenen Bedeutungen besitzen,

oder ein reaktionsfähiges Derivat davon, mit einem Hydrazinderivat der Formel

$$H_2N-R \qquad III$$

worin R die oben angegebenen Bedeutungen besitzt, umsetzt,

b) ein Hydrazid der Formel

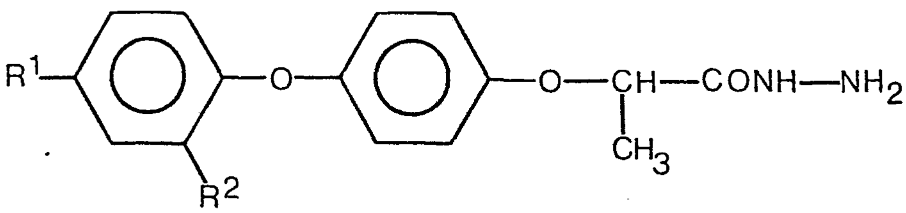

IV

worin $R^1$ und $R^2$ die oben angegebenen Bedeutungen besitzen,

mit einer Säure der Formel

$$HO-\overset{X \atop \|}{C}-Y-R^5 \qquad Va$$

$$HO-CO-R^8 \qquad Vb$$

$$HO-SO_2-R^9 \qquad Vc$$

worin $R^5$, X, Y, $R^8$ und $R^9$ die oben angegebenen Bedeutungen besitzen,

oder einem reaktionsfähigen Derivat davon, umsetzt,

c) eine Dithiocarbazinsäure der Formel

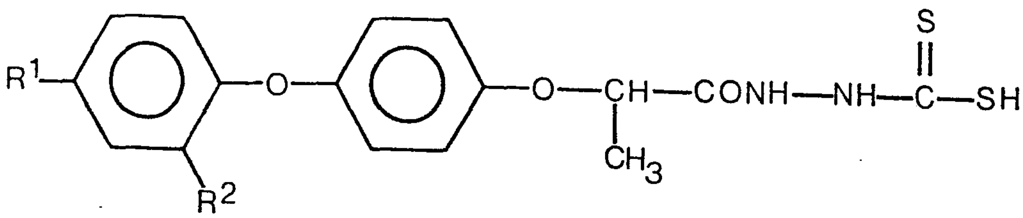

VI

worin $R^1$ und $R^2$ die oben angegebenen Bedeutungen besitzen,

oder ein Alkalimetall- oder Erdalkalimetallsalz davon, mit einer Verbindung der Formel

$$R^5-Z^1 \qquad VII$$

worin $R^5$ die oben angegebenen Bedeutungen besitzt und $Z^1$ eine Abgangsgruppe bedeutet,

umsetzt,

d) ein Dithiocarbazat der Formel

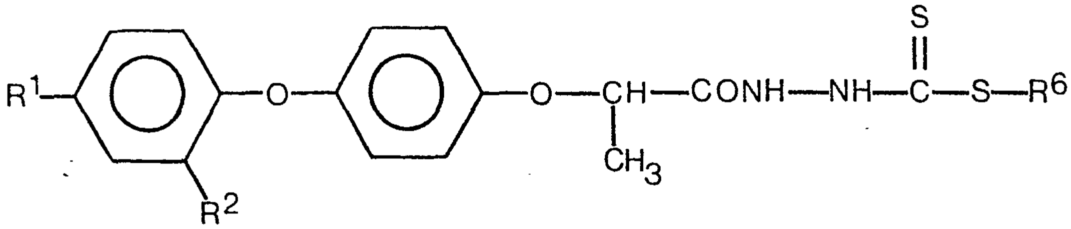

VIII

worin $R^1$, $R^2$ und $R^6$ die oben angegebenen Bedeutungen besitzen,

oder ein Alkalimetall- oder Erdalkalimetallsalz davon, mit

einer Verbindung der Formel

$$R^7Z^1 \qquad IX$$

worin $R^7$ und $Z^1$ die oben angegebenen Bedeutungen besitzen,

umsetzt,

e) ein Hydrazid der Formel IV, wie oben definiert, mit einem Aldehyd bzw. Keton der Formel

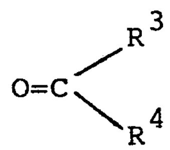

X

worin $R^3$ und $R^4$ die oben angegebenen Bedeutungen besitzen,

umsetzt, oder

f) ein Phenol der Formel

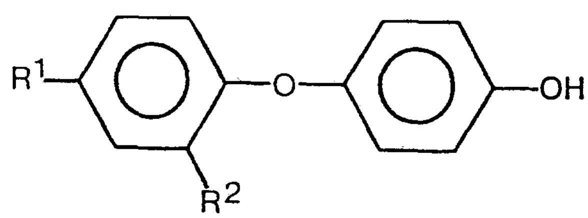

XI

worin $R^1$ und $R^2$ die oben angegebenen Bedeutungen besitzen,

oder ein Alkalimetallsalz davon, mit einem Hydrazinderivat der Formel

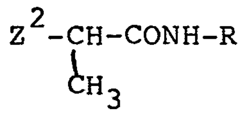

XII

worin R die oben angegebenen Bedeutungen besitzt, und $Z^2$ eine Abgangsgruppe bedeutet,

umsetzt.

13. Verfahren zur Herstellung eines Unkrautbekämpfungsmittels, dadurch gekennzeichnet, dass man mindestens eine der in den Ansprüchen 1 bis 3, 6 und 10 genannten Verbindungen mit Formulierungshilfsstoffen vermischt.

14. Verfahren zur Bekämpfung von Unkräutern, dadurch gekennzeichnet, dass man das gegen Unkräuter zu schützende Gut und/oder die Unkräuter mit einer wirksamen Menge einer bzw. eines der in den Ansprüchen 1 bis 3, 6 und 10 genannten Verbindungen bzw. Mittel behandelt.

15. Verwendung einer bzw. eines der in den Ansprüchen 1 bis 3, 6 und 10 genannten Verbindungen bzw. Mittel zur Bekämpfung von Unkräutern.

***